(19) **Europäisches Patentamt / European Patent Office / Office européen des brevets**

(11) **EP 2 005 925 B1**

(12) **EUROPEAN PATENT SPECIFICATION**

(45) Date of publication and mention
of the grant of the patent:
**08.08.2012 Bulletin 2012/32**

(21) Application number: **07740399.6**

(22) Date of filing: **29.03.2007**

(51) Int Cl.:
*A61F 13/02* (2006.01)   *A61L 15/58* (2006.01)
*B32B 27/40* (2006.01)   *B32B 27/12* (2006.01)
*A61L 15/26* (2006.01)

(86) International application number:
**PCT/JP2007/056961**

(87) International publication number:
**WO 2007/114295 (11.10.2007 Gazette 2007/41)**

(54) **MEDICAL SHEET BASE AND MEDICAL SHEET COMPRISING THE SAME**

MEDIZINISCHES BASIS-FLÄCHENGEBILDE UND DIESES ENTHALTENDES MEDIZINISCHES FLÄCHENGEBILDE

BASE DE DRAP D'EXAMEN MÉDICAL ET DRAP D'EXAMEN MÉDICAL LA CONTENANT

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **31.03.2006 JP 2006096914**

(43) Date of publication of application:
**24.12.2008 Bulletin 2008/52**

(73) Proprietors:
• **KB Seiren, Ltd.**
**Sabae-shi**
**Fukui 916-0038 (JP)**
• **SEIREN CO., LTD.**
**Fukui-shi,**
**Fukui-ken 918-8560 (JP)**

(72) Inventors:
• **TANAKA, Yutaka**
**Osaka-shi, Osaka 5300001 (JP)**

• **SHIMIZU, Hiroyasu**
**Sabae-shi, Fukui 9160038 (JP)**
• **TAKAHIRA, Sunao**
**Osaka-shi, Osaka 5300001 (JP)**
• **HIROTA, Makoto**
**Fukui 9188560 (JP)**

(74) Representative: **Isarpatent**
**Patent- und Rechtsanwälte**
**Postfach 44 01 51**
**80750 München (DE)**

(56) References cited:
EP-A2- 1 541 106        WO-A1-99/48684
JP-A- 4 317 654         JP-A- 2004 202 923
JP-A- 2004 315 817      JP-A- 2005 178 365
JP-A- 2005 538 873      US-A- 5 584 801
US-A1- 2005 106 980

EP 2 005 925 B1

**Description**

TECHNICAL FIELD

The present invention relates to a medical sheet base including a polyurethane nonwoven fabric and a polyurethane film combined with each other and excellent in stretchability, air permeability, moisture permeability, water resistance and flexibility, and to a medical sheet including the medical sheet base.

BACKGROUND ART

Exemplary materials employed for prior-art medical sheet bases include an air- and moisture-permeable material prepared by punching a moisture-impermeable film, a material utilizing a nonporous and moisture-permeable polyurethane film, and a composite material prepared by combining any of the aforementioned film materials with a fiber material such as a nonwoven or knitted fabric (see, for example, Patent Document 1).

However, the prior-art medical sheet bases suffer from the following drawbacks. The material prepared by punching the moisture-impermeable film has a problem associated with water resistance such as infiltration of water through punch holes. Further, a portion of the film present between the punch holes is moisture- and air-impermeable. Therefore, this material is liable to cause partial dampness and sometimes itchiness when being used as a medical sheet.

On the other hand, the material utilizing the nonporous and moisture-permeable polyurethane film is water-resistant, and moisture-permeable to some extent. However, this material is also liable to cause dampness and itchiness with still insufficient moisture permeability and air impermeability.

Patent Document 1: JP-A-8(1996)-33673

JP 2004 202923 A discloses a laminate laminating a urethane film and a non-woven fabric which has moisture permeability, excellent transparency and workability as a product as well as flexibility.

DISCLOSURE OF THE INVENTION

PROBLEMS TO BE SOLVED BY THE INVENTION

It is an object of the present invention to provide a medical sheet base which is improved in stretchability, air permeability, moisture permeability, water resistance and flexibility over the prior-art medical sheet bases as required of the medical sheet base for ensuring conformable use thereof, and to provide a medical sheet including the medical sheet base.

MEANS FOR SOLVING THE PROBLEMS

To achieve the aforementioned object, there is provided a medical sheet base comprising a polyurethane nonwoven fabric and a polyurethane film combined with each other, wherein the polyurethane nonwoven fabric comprises continuous filaments each having an average filament diameter of not greater than 50 $\mu$m, the filaments being deposited in a substantially unbound state and bonded to each other, wherein the polyurethane film has minute pores present in opposite surfaces thereof and each having a diameter of not greater than 10 $\mu$m and being spaced an average distance of not greater than 10 $\mu$m from each other, and minute pores communicating with each other along the thickness thereof, and the polyurethane film has an air permeability of 0.01 to 2.0 cc/cm$^2$·sec, a moisture permeability of not less than 10000 g/m$^2$·24hr, and a water resistance pressure of not less than 500 mmH$_2$O.

In the present invention, the polyurethane nonwoven fabric and the polyurethane film are preferably combined with each other by using an adhesive, and the adhesive is preferably applied to parts of the polyurethane nonwoven fabric and the polyurethane film.

In the present invention, the medical sheet base preferably has a recovery ratio of not less than 70% as measured after the medical sheet base is stretched by 100%, an air permeability of 0.01 to 1.0 cc/cm$^2$·sec, a moisture permeability of not less than 5000 g/m$^2$·24hr, and a bending resistance of not greater than 60 mm.

In the present invention, the medical sheet base preferably has an air permeability of 0.015 to 1.5 cc/cm$^2$·sec as measured when the medical sheet base is stretched by 100%.

In order to achieve the object of the present invention, there is further provided a medical sheet comprising the aforementioned medical sheet base.

EFFECTS OF THE INVENTION

The present invention provides the medical sheet base comprising the specific polyurethane nonwoven fabric and the specific polyurethane film. With this arrangement, the medical sheet base is excellent in stretchability, air permeability, moisture permeability, water resistance and flexibility.

The medical sheet comprising the medical sheet base according to the present invention is free from dampness of skin even if being applied to the skin at a higher temperature for a long period of time. Further, the medical sheet is free from separation and lifting from skin even if being applied to a joint or other portion of a human body which requires stretchability. Thus, the medical sheet provides excellent effects.


BEST MODE FOR CARRYING OUT THE INVENTION

A nonwoven fabric to be used in the present invention is composed of a polyurethane excellent in stretchability and recoverability.

The polyurethane is prepared by polymerizing a high molecular weight polyol compound serving as a soft segment, a lower molecular weight diol compound serving as a hard segment and an organic diisocyanate compound.

The higher molecular weight polyol compound is a diol polymer compound having a molecular weight of 500 to 6000 and prepared by polycondensation, addition polymerization (e.g., ring opening polymerization) or polyaddition. Typical examples of the diol polymer compound include polyester diols, polyether diols and polycarbonate diols, and condensation copolymerization products (e.g., polyesterether diols) of any of these diols. These diol polymer compounds may be used either alone or in combination.

Preferred examples of the polyester diols include saturated polyester diols obtained by reactions between a $C_2$ to $C_{10}$ alkanediol compound such as propylene glycol, 1,4-butanediol, 1,5-pentanediol, 3-methyl-1,5-pentanediol, 1,6-hexanediol, neopentyl glycol or 2-methylpropanediol or a mixture of any of these alkanediol compounds and a $C_4$ to $C_{12}$ aliphatic or aromatic dicarboxylic acid such as glutaric acid, adipic acid, pimelic acid, suberic acid, sebacic acid, terephthalic acid or isophthalic acid or a mixture of any of these acids, and polylactone diols such as polycaprolactone glycol and polyvalerolactone glycol. Preferred examples of the polyether diols include polyalkylene ether diols such as polyethylene ether glycols, polypropylene ether glycols, polytetramethylene ether glycols and polyhexamethylene ether glycols.

The lower molecular weight diol compound is a diol compound having a molecular weight of less than 500. Examples of such a diol compound include aliphatic and aromatic diols such as ethylene glycol, propylene glycol, 1,4-butanediol, 1,5-pentane glycol, 3-methylpentane glycol, 1,6-hexanediol and 1,4-bishydroxyethylbenzene. These lower molecular weight diol compounds may be used either alone or in combination.

Examples of the organic diisocyanate compound include aliphatic and aromatic diisocyanate compounds such as 4,4'-diphenylmethane diisocyanate, tolylene diisocyanate, isophorone diisocyanate, hydrogenated diphenylmethane diisocyanate, xylylene diisocyanate, 2,6-diisocyanatemethyl caproate and hexamethylene diisocyanate. These organic diisocyanate compounds may be used either alone or in combination.

As required, an antioxidant such as of a hindered phenol or an amine, a UV absorber such as of benzotriazole or a hindered amine, a lubricating agent such as an amide wax or a montanic acid wax, a hydrolysis preventing agent such as a carbodiimide compound, a pigment such as titanium oxide or red iron oxide, a gas yellowing preventing agent and other additives for polymers may be added.

Exemplary polymerization methods include a continuous melt polymerization method, a kneader method, a bat curing method and a belt method. In the present invention, any of these polymerization methods may be used.

For production of the polyurethane nonwoven fabric to be used in the present invention, a known production method such as a spun-bonding method or a melt-blowing method may be employed. Among these methods, the melt-blowing method is preferred in consideration of the stretchability and the flexibility of the resulting nonwoven fabric.

An ordinary melt-blowing method will be described below. The thermoplastic polyurethane is melted in an extruder, and the melted polymer is metered by a gear pump and ejected from spinning nozzle holes arranged in line. Gas heated to a high temperature is ejected at a high speed from slits disposed on opposite sides of the nozzle holes, and the polymer ejected from the nozzle holes is thinned and cooled by high speed gas streams. Thus, continuous filaments are formed. The thin filaments are separated from the gas streams in a substantially unbound state on a collecting device on a moving conveyor net, and deposited on the net. The filaments are fusion-bonded to each other at contact points in the deposited state by their heat.

In the polyurethane nonwoven fabric to be used in the present invention, the filaments should be deposited in a longitudinally spread and substantially unbound state. If the filaments are fusion-bonded in a bound and unspread state, the resulting nonwoven fabric has poorer uniformity and significantly reduced flexibility.

The polyurethane nonwoven fabric to be used in the present invention should have an average filament diameter of not greater than 50 μm, more preferably 5 to 30 μm, in consideration of practical properties such as flexibility and texture.

Further, the polyurethane nonwoven fabric preferably has a per unit area weight of about 20 to about 300 $g/m^2$. If the per unit area weight is less than 20 $g/m^2$, the nonwoven fabric is likely to have lower strength, suffering from problems in practice. If the per unit area weight is greater than 300 $g/m^2$, the nonwoven fabric is likely to have a harder texture with poorer stretchability and flexibility, failing to ensure comfortable use in practice.

The polyurethane nonwoven fabric preferably has a breaking elongation of not less than 200%, more preferably not less than 300%, and a breaking strength of not less than 200 cN/cm, more preferably not less than 250 cN/cm. If the breaking elongation is less than 200%, a medical sheet including the polyurethane nonwoven fabric is likely to be stiff with insufficient stretchability when being applied to a joint or other portion which requires stretchability. In order to ensure the comfortable use, the breaking elongation is preferably not less than 300%. If the breaking strength is less than 200 cN/cm, the medical sheet base is liable to tear during processing or during use.

The polyurethane nonwoven fabric preferably has a recovery ratio of not less than 70%, more preferably not less than 80%, as measured after the nonwoven fabric is stretched by 100%. If the recovery ratio is within this range, the nonwoven fabric is excellent in elongation recoverability.

The polyurethane nonwoven fabric preferably has an air permeability of 10 to 500 $cc/cm^2 \cdot sec$. If the air permeability is less than 10 $cc/cm^2 \cdot sec$, the polyurethane nonwoven fabric is likely to give uncomfortable feeling such as dampness or itchiness to a user when being used for a medical sheet. If the air permeability is greater than 500 $cc/cm^2 \cdot sec$, the flexibility and the recoverability of the nonwoven fabric are likely to be reduced.

The polyurethane nonwoven fabric preferably has a bending resistance of not greater than 50 mm. If the bending resistance is greater than 50 mm, the user is likely to have unnatural feeling when the polyurethane nonwoven fabric is used for a medical sheet.

On the other hand, the polyurethane film to be used in the present invention has minute pores present in opposite surfaces thereof and each having a diameter of not greater than 10 $\mu$m, and minute pores communicating with each other along the thickness thereof. The pores are preferably spaced an average distance of not greater than 10 $\mu$m from each other. With this arrangement, the polyurethane film is excellent in air permeability, moisture permeability and water resistance. If the diameter of each of the pores is greater than 10 $\mu$m, the air permeability and the moisture permeability are improved, but the water resistance is likely to be reduced. If the average distance between the pores is greater than 10 $\mu$m, the moisture permeability is reduced. Therefore, the polyurethane film is likely to give uncomfortable feeling such as dampness or itchiness to the user when being used for a medical sheet.

For example, a polyurethane film disclosed in JP-A-2004-315817 titled "Polyurethane Film and Production Method for the Film" may be used as the polyurethane film in the present invention. More specifically, a polyurethane solution is prepared by dissolving polyurethane in a solvent mixture of a selected organic solvent and water, and casted on a releasable sheet. At the initial stage of a drying step, the organic solvent is selectively evaporated, whereby the proportion of the water in the polyurethane solution is increased. Thus, the surface tension of the polyurethane solution is increased to be repelled on the releasable sheet. Further, the polyurethane and the water are separated from each other on the releasable sheet with the proportion of the water in the polyurethane solution being increased, whereby minute pores are formed. In the final stage of the drying step, the remaining water is evaporated. Thus, a porous polyurethane film is provided.

The polyurethane film desirably has an air permeability of 0.01 to 2.0 $cc/cm^2 \cdot sec$, a water resistant pressure of not less than 500 $mmH_2O$, and a breaking elongation of not less than 200%. If the air permeability is less than 0.01 $cc/cm^2 \cdot sec$, the polyurethane film is likely to give uncomfortable feeling such as dampness or itchiness to the user with insufficient air permeability when being used for a medical sheet. If the air permeability is greater than 2.0 $cc/cm^2 \cdot sec$, the polyurethane film has insufficient water resistance with an excessively great number of pores, so that water penetration will easily occur during use. If the breaking elongation is less than 200%, a medical sheet including the polyurethane film is likely to be stiff with insufficient elongation when being applied to a joint or other portion which requires stretchability. If the water resistant pressure is less than 500 $mmH_2O$, the polyurethane film is insufficient in water resistance and, when the polyurethane film is used for a medical sheet, water penetration will easily occur during use.

The polyurethane film desirably has a thickness of 5 to 15 $\mu$m. If the thickness is less than 5 $\mu$m, the film has lower strength, and is liable to tear when being used for the medical sheet. If the thickness is greater than 15 $\mu$m, the film is likely to have a hard texture to give unnatural feeling to the user.

The inventive medical sheet base includes the polyurethane nonwoven fabric and the polyurethane film combined with each other, and preferably has a breaking elongation of not less than 200%. Further, the inventive medical sheet base preferably has a recovery ratio of not less than 70% as measured after the medical sheet base is stretched by 100%, an air permeability of 0.01 to 1.0 $cc/cm^2 \cdot sec$, a moisture permeability of not less than 5000 $g/m^2 \cdot 24hr$, and a bending resistance of not greater than 60 mm. If the breaking elongation is less than 200%, a medical sheet including the medical sheet base is likely to be stiff with insufficient elongation when being applied to a joint or other portion which requires stretchability. If the 100%-elongation recovery ratio is less than 70%, a medical sheet including the medical sheet base is liable to give unnatural feeling to the user with insufficient recoverability when being applied to a joint or other portion which requires stretchability. If the air permeability is less than 0.01 $cc/cm^2 \cdot sec$, the medical sheet base is likely to give uncomfortable feeling such as dampness or itchiness to the user with insufficient air permeability when being used for a medical sheet. On the other hand, if the air permeability is greater than 1.0 $cc/cm^2 \cdot sec$, the water resistance is insufficient with an excessively great number of pores, so that water penetration will easily occur during use. If the moisture permeability is less than 5000 g/m$^2$·24hr, the medical sheet base is likely to give uncomfortable feeling such as dampness or itchiness to the user with insufficient moisture permeability when being used as a medical sheet. If the bending resistance is greater than 60 mm, the medical sheet base is likely to give unnatural feeling to the user when being used as a medical sheet.

The medical sheet base satisfying the aforementioned conditions is excellent in stretchability, air permeability, moisture permeability, water resistance and flexibility, and can be used as an excellent medical sheet.

The inventive medical sheet base preferably has an air permeability of 0.015 to 1.5 cc/cm$^2$·sec as measured when the medical sheet base is stretched by 100%. Further, the inventive medical sheet base preferably has a water resistant pressure of not less than 600 mmH$_2$O. The medical sheet base is air-permeable even if being stretched. Therefore, the inventive medical sheet base is excellent in air and moisture permeability even if being used in a stretched state and, therefore, relieves the user from the dampness and the itchiness.

Exemplary methods for combining the polyurethane nonwoven fabric and the polyurethane film include a method using an adhesive for bonding the polyurethane nonwoven fabric to the polyurethane film, and a method employing fusion-bonding, but the method using the adhesive is preferred.

In the method using the adhesive, an adhesive application amount and adhesive application positions are controlled according to the thickness and the surface state of the nonwoven fabric for selection of optimum bonding conditions. Thus, a composite material having well-balanced bonding strength, stretchability and air permeability can be easily provided.

Usable as the adhesive is an aqueous adhesive, a solvent type adhesive or a hot-melt type adhesive. For chemical composition, a urethane adhesive and an acryl adhesive are preferred. Particularly, the hot-melt type urethane adhesive is preferred.

Where the hot-melt type adhesive is used, the adhesive is applied to the polyurethane nonwoven fabric and/or the polyurethane film, which are in turn bonded to each other, and then cooled to a temperature not higher than the melting point of the adhesive to be thereby cured. Thus, the nonwoven fabric is prevented from being excessively impregnated with the adhesive. Where the aqueous or solvent type adhesive is used, on the other hand, the adhesive is liable to intrude into voids of the nonwoven fabric and into the minute pores of the film after the application of the adhesive, thereby adversely affecting the air permeability and the moisture permeability.

Further, the urethane adhesive which is stretchable is preferred for prevention of reduction in the stretchability of the composite medical sheet base.

The adhesive is preferably applied to parts of the polyurethane nonwoven fabric and/or the polyurethane film by a gravure coating method or a printing method in order to ensure the air permeability and the moisture permeability of the composite medical sheet base.

Therefore, the best method for combining the polyurethane nonwoven fabric and the polyurethane film is to apply a hot-melt type urethane adhesive to parts of the polyurethane nonwoven fabric by means of a hot gravure roll and combine the polyurethane nonwoven fabric with the polyurethane film.

A medical sheet according to the present invention includes the aforementioned sheet base alone, but may further include a moisture absorbing sheet and/or a releasable sheet provided, as required, on a surface of the medical sheet base to which an adhesive is applied. The adhesive to be used in this case is not particularly limited as long as it has a pressure sensitive adhesion property at an ordinary temperature. Examples of the pressure-sensitive adhesive include acryl adhesives, rubber adhesives, silicone adhesives and polyurethane adhesives. A known moisture absorbing sheet may be used as the moisture absorbing sheet in the present invention. Examples of the moisture absorbing sheet include sterilized woven, knitted and nonwoven fabrics such as of cotton fibers and cellulose fibers. The moisture absorbing sheet typically has a smaller size than the medical sheet base. The position of the moisture absorbing sheet on the medical sheet base is not particularly limited, but may be a center portion or an edge portion of the medical sheet.

The releasable sheet is not particularly limited, as long as it can be easily separated from a layer of the pressure-sensitive adhesive. Examples of the releasable sheet include a paper sheet, a fabric or a plastic film coated with a silicone releasing agent.

The present invention will hereinafter be described more specifically by way of examples, but it should be understood that the invention be not limited to these examples. In the inventive examples and comparative examples, characteristic property values and evaluation values were determined by the following measurement methods and evaluation methods.

(1) Breaking Strength and Elongation

In conformity with JIS L-1096, a 2-cm wide sample was stretched at a stretching rate of 10 cm/min with a gripping distance of 5 cm, and the strength per centimeter width and the elongation were determined at breakage.

(2) 100% Elongation Recovery Ratio

A 2-cm wide sample was stretched at a stretching rate of 10 cm/min with a gripping distance of 5 cm by 100% with respect to the gripping distance and, immediately thereafter, restored to an original length at the same rate. Based on a recorded load-elongation curve, a residual elongation ratio D (%) was determined. Then, a 100% elongation recovery ratio was calculated from the following expression:

$$\texttt{100\% elongation recovery ratio (\%) = 100 - D}$$

(3) Air Permeability

JIS L-1096A (Fragile type method).

(4) Bending Resistance

JIS L-1096A (45-degree cantilever method).

(5) States of Opposite Surfaces and Section of Film

A front surface, a back surface (in contact with a releasable sheet) and a section of a film were observed with the use of a scanning electron microscope.

(6) Moisture Permeability

JIS L-1099 (A-1 method).

(7) Water Resistance (Water Resistant Pressure)

JIS L-1092A method (lower water pressure method) or JIS L-1092B method (higher water pressure method).
For a film or a medical sheet base having a water resistant pressure of lower than 1000 mmH$_2$O, a lower water pressure method was employed.

EXAMPLES

Example 1

A polybutylene adipate (PBA) having a molecular weight of 2000, 4,4'-diphenylmethane diisocyanate (MDI) and 1,4-butanediol (BD) were mixed in a polymerization ratio of PBA/MDI/BD = 80/59/17.5, and mass-polymerized through one shot by means of a twin screw extruder continuous polymerization apparatus. Thus, a thermoplastic polyurethane having a Shore A hardness of 95 was prepared. The thermoplastic polyurethane was dried in vacuum at a temperature of 80°C for 24 hours. The thermoplastic polyurethane had a moisture content of 100 ppm as measured by the Carl Fischer method.
The thermoplastic polyurethane was melt-kneaded at 230°C by means of an extruder, metered by a gear pump, and ejected from melt-blowing nozzles aligned in line at a pitch of 2 mm and each having a hole having a diameter of 0.5 mm. The polymer was spun at a spinning rate of 0.15 g/min per nozzle hole, and thinned and solidified by hot air blown (at 238°C at 9 NL/cm/min) from opposite sides of the nozzles, whereby filaments each having an average filament diameter of 25 $\mu$m were formed and blown onto a moving conveyor net. Thus, an elastic polyurethane filament nonwoven fabric having a per unit area weight of 50 g/m$^2$ was provided. At this time, the spinning temperature was 235°C. In the nonwoven fabric, the elastic polyurethane monofilaments had entanglement points fusion-bonded to each other. The physical property values of the nonwoven fabric are as follows.

Physical Property Values of Elastic Polyurethane Filament Nonwoven Fabric

Average filament diameter: 25 $\mu$m

Per unit area weight: 50 g/m$^2$
Breaking strength: 294 cN/cm
Breaking elongation: 540%
100% elongation recovery ratio: 92%
Air permeability: 400 cc/cm$^2$·sec (for correction)
Bending resistance: 20 mm

For a polyurethane film, on the other hand, a polyurethane mixture solution was prepared according to the following formulation.

Formulation

100 parts by weight of HIMUREN X-3040 (a polyurethane resin solution available from Dainichiseika Color & Chemicals Mfg. Co., Ltd.)

1. 0 part by weight of RESAMINE X-100 (a crosslinking agent available from Dainichiseika Color & Chemicals Mfg. Co., Ltd.)
60 parts by weight of a methyl ethyl ketone/toluene mixture (mixing ratio of 1:2)
70 parts by weight of a methyl ethyl ketone/water mixture (mixing ratio of 1:10)

Then, the polyurethane mixture solution was casted on a releasable sheet to a thickness of 100 μm by means of a knife coater and, immediately thereafter, dried at 70°C for 1 minute and then at 130°C for 1 minute. Thus, a polyurethane film having a thickness of 11 μm was prepared on the releasable sheet.

Observation of the film indicated that the film had minute pores generally uniformly formed at an average spacing of 7 μm on opposite surfaces thereof as each having a diameter of not greater than 10 μm and minute pores communicating with each other along the thickness thereof as seen in section. The physical property values of the polyurethane film are as follows.

Physical Property Values of Polyurethane Film

Air permeability: 1.5 cc/cm$^2$·sec
Moisture permeability: 12100 g/m$^2$·29hr
Water resistant pressure: 1100 mmH$_2$O

The polyurethane nonwoven fabric was combined with the polyurethane film prepared on the releasable sheet in the following manner. Immediately after a urethane hot-melt adhesive (TYFORCE NH-300 available from Dainippon Ink & Chemicals, Inc.) was applied in a dot pattern to the polyurethane nonwoven fabric by means of a gravure coater heated to a temperature of 110°C, the resulting polyurethane nonwoven fabric was bonded to a polyurethane film surface of the polyurethane film prepared on the releasable sheet. After the resulting product was aged at 25°C for two days, the releasable sheet was peeled off. Thus, a medical sheet base was produced.

Example 2

A polyurethane film having a thickness of 15 μm was prepared in substantially the same manner as in Example 1, except that the polyurethane mixture solution was casted to a thickness of 150 μm on a releasable sheet. Observation of the film indicated that the film had minute pores generally uniformly formed at an average spacing of 7 μm on opposite surfaces thereof as each having a diameter of not greater than 10 μm and minute pores communicating with each other along the thickness thereof as seen in section. The physical property values of the polyurethane film are as follows.

Physical Property Values of Polyurethane Film

Air permeability: 0.8 cc/cm$^2$·sec
Moisture permeability: 11000 g/m$^2$·24hr
Water resistant pressure: 2240 mmH$_2$O

Then, a medical sheet base was produced in substantially the same manner as in Example 1, except that the polyurethane film thus prepared was used.

Example 3

A polyurethane film having a thickness of 6 $\mu$m was prepared in substantially the same manner as in Example 1, except that the methyl ethyl ketone/toluene mixture (mixing ratio of 1:2) and the methyl ethyl ketone/water mixture (mixing ratio of 1:10) were used in proportions of 55 parts by weight and 75 parts by weight, respectively, for the formulation of the polyurethane mixture solution and the polyurethane resin solution was casted to a thickness of 50 $\mu$m on a releasable sheet by means of a knife coater. Observation of the film indicated that the film had minute pores generally uniformly formed at an average spacing of 6 $\mu$m on opposite surfaces thereof as each having a diameter of not greater than 10 $\mu$m and minute pores communicating with each other along the thickness thereof as seen in section. The physical property values of the polyurethane film are as follows.

Physical Property Values of Polyurethane Film

Air permeability: 1.8 cc/cm$^2$·sec
Moisture permeability: 13200 g/m$^2$·24hr
Water resistant pressure: 550 mmH$_2$O

Then, a medical sheet base was produced in substantially the same manner as in Example 1, except that the polyurethane film thus prepared and the elastic polyurethane filament nonwoven fabric prepared in Example 1 as having a per unit area weight of 50 g/cm$^2$ were used.

Example 4 (comparative)

A polyurethane film having a thickness of 3 $\mu$m was prepared in substantially the same manner as in Example 1, except that the methyl ethyl ketone/toluene mixture (mixing ratio of 1:2) and the methyl ethyl ketone/water mixture (mixing ratio of 1:10) were used in proportions of 50 parts by weight and 80 parts by weight, respectively, for the formulation of the polyurethane mixture solution and the polyurethane resin solution was casted to a thickness of 30 $\mu$m on a releasable sheet by means of a knife coater. Observation of the film indicated that the film had minute pores generally uniformly formed at an average spacing of 5 $\mu$m on opposite surfaces thereof as each having a diameter of not greater than 10 $\mu$m and minute pores communicating with each other along the thickness thereof as seen in section. The physical property values of the polyurethane film are as follows.

Physical Property Values of Polyurethane Film

Air permeability: 2.5 cc/cm$^2$·sec
Moisture permeability: 13600 g/m$^2$·24hr
Water resistant pressure: 260 mmH$_2$O

Then, a medical sheet base was produced in substantially the same manner as in Example 1, except that the polyurethane film thus prepared and the elastic polyurethane filament nonwoven fabric prepared in Example 1 as having a per unit area weight of 50 g/cm$^2$ were used.

Comparative Example 1

A medical sheet base was produced in substantially the same manner as in Example 1, except that a nonporous polyurethane film prepared by using a polyurethane mixture solution having the following formulation was used as the polyurethane film to be bonded.

Formulation

100 parts by weight of HIMUREN NPU-5 (a polyurethane resin solution available from Dainichiseika Color & Chemicals Mfg. Co., Ltd.)

25 parts by weight of isopropyl alcohol
25 parts by weight of toluene

The polyurethane resin solution was casted to a thickness of 100 µm on a releasable sheet by means of a knife coater and, immediately thereafter, dried at 130°C for 1 minute. Thus, the nonporous polyurethane film was prepared on the releasable sheet as having a thickness of 10 µm.

Comparative Example 2

A polyurethane film having a thickness of 11 µm was prepared in substantially the same manner as in Example 1, except that 20 parts by weight of an aqueous polyurethane resin (ELASTRON W-11P available from Dai-ichi Kogyo Seiyaku Co., Ltd.) was additionally used for the formulation of the polyurethane of Example 1. Observation of the film indicated that the film had minute pores generally uniformly formed at an average spacing of 7 µm on opposite surfaces thereof as each having a diameter of 11 to 15 µm and minute pores communicating with each other along the thickness thereof as seen in section. The physical property values of the polyurethane film are as follows.

Physical Property Values of Polyurethane Film

Air permeability: 2.8 cc/cm$^2$·sec
Moisture permeability: 13900 g/m$^2$·24hr
Water resistant pressure: 230 mmH$_2$O

Then, a medical sheet base was produced in substantially the same manner as in Example 1, except that the polyurethane film thus prepared and the elastic polyurethane filament nonwoven fabric prepared in Example 1 as having a per unit area weight of 50 g/cm$^2$ were used.

Comparative Example 3

A thermoplastic polyurethane having a Shore A hardness of 98 was prepared in substantially the same manner as in Example 1, except that a polybutylene adipate (PBA) having a molecular weight of 2000, 4,4'-diphenylmethane diisocyanate (MDI) and 1,4-butanediol (BD) were mixed in a polymerization ratio of PBA/MDI/BD = 78/60/18. As in Example 1, the thermoplastic polyurethane was ejected from the melt-blowing nozzles. Thus, an elastic polyurethane filament nonwoven fabric having an average filament diameter of 54 µm was provided. The physical property values of the elastic polyurethane filament nonwoven fabric are as follows.

Physical Property Values of Elastic Polyurethane Filament Nonwoven Fabric

Average filament diameter: 54 µm
Per unit area weight: 50 g/m$^2$
Breaking strength: 310 cN/cm
Breaking elongation: 500%
100% elongation recovery ratio: 80%
Air permeability: 510 cc/cm$^2$·sec
Bending resistance: 60 mm

Then, a medical sheet base was produced in substantially the same manner as in Example 1, except that the elastic polyurethane filament nonwoven fabric thus prepared and the polyurethane film prepared in Example 1 were used.
The physical property values of the medical sheet bases of Examples 1 to 4 and Comparative Examples 1 to 3 are collectively shown in Table 1.

Table 1

| | | Breaking elongation (%) | 100% elongation recovery ratio (%) | Air permeability (cc/cm$^2$·sec) | Air permeability at 100% elongation (cc/cm$^2$·sec) | Moisture permeability (g/m$^2$·24hr) | Bending resistance (mm) | Water resistant pressure (mmH$_2$O) | |
|---|---|---|---|---|---|---|---|---|---|
| | | | | | | | | High pressure method | Low pressure method |
| Example 1 | Polyurethane nonwoven fabric/porous polyurethane film | 420 | 75 | 0.20 | 0.24 | 11200 | 26 | 1230 | - |
| Example 2 | Polyurethane nonwoven fabric/porous polyurethane film | 405 | 78 | 0.05 | 0.06 | 10500 | 31 | 2350 | - |
| Example 3 | Polyurethane porous polyurethane film | 436 | 75 | 0.80 | 0.95 | 12800 | 26 | < 1000 | 640 |
| Example 4 | Polyurethane nonwoven fabric/porous polyurethane film | 442 | 74 | 1.2 | 1.4 | 13200 | 25 | < 1000 | 300 |
| Comparative Example 1 | Polyurethane nonwoven fabric / nonporous polyurethane film | 424 | 75 | 0 | 0 | 3800 | 30 | 3500 | - |

(continued)

| | | Breaking elongation (%) | 100% elongation recovery ratio (%) | Air permeability (cc/cm$^2$·sec) | Air permeability at 100% elongation (cc/cm$^2$·sec) | Moisture permeability (g/m$^2$·24hr) | Bending resistance (mm) | Water resistant pressure (mmH$_2$O) | |
|---|---|---|---|---|---|---|---|---|---|
| | | | | | | | | High pressure method | Low pressure method |
| Comparative Example 2 | Polyurethane nonwoven fabric/porous polyurethane film | 401 | 74 | 1.4 | 1.7 | 13000 | 25 | < 1000 | 290 |
| Comparative Example 3 | Polyurethane nonwoven fabric/porous polyurethane film | 360 | 68 | 0.20 | 0.24 | 11500 | 63 | 1250 | - |

As can be understood from the above results, the medical sheet bases of Examples are excellent in stretchability, air permeability, moisture permeability, water resistance and flexibility.

On the other hand, the medical sheet base of Comparative Example 1 is insufficient in moisture permeability and air permeability. Therefore, where the medical sheet base is used as a medical sheet, it is expected that the medical sheet gives uncomfortable feeling such as dampness to a user. The medical sheet base of Comparative Example 2 is insufficient in air permeability and water resistance, and inconvenient to use. Further, the medical sheet base of Comparative Example 3 has an insufficient 100%-elongation recovery ratio and an excessively high bending resistance and, therefore, is inconvenient to use.

INDUSTRIAL APPLICABILITY

The medical sheet base and the medical sheet according to the present invention are excellent in stretchability, air permeability, moisture permeability, water resistance and flexibility. The inventive medical sheet base is much more stretchable, air-permeable and moisture-permeable than the prior-art medical sheet bases. Even if the medical sheet base is applied as a medical sheet to skin at a higher temperature for a longer period of time, the skin is free from dampness. Further, even if the medical sheet base is applied as a medical sheet to a joint or other portion of a human body which requires stretchability, the medical sheet is free from separation and lifting from the skin. Thus, the medical sheet provides excellent effects. Specifically, the inventive medical sheet can be advantageously used for a medical adhesive tape, an adhesive bandage, a drug patch or the like.

**Claims**

1.  A medical sheet base comprising a polyurethane nonwoven fabric and a polyurethane film combined with each other, wherein the polyurethane nonwoven fabric comprises continuous filaments each having an average filament diameter of not greater than 50 $\mu$m, the filaments being deposited in a substantially unbound state and bonded to each other, wherein the polyurethane film has minute pores present in opposite surfaces thereof and each having a diameter of not greater than 10 $\mu$m and being spaced an average distance of not greater than 10 $\mu$m from each other, and minute pores communicating with each other along a thickness thereof, and the polyurethane film has an air permeability of 0.01 to 2.0 cc/cm$^2$·sec, a moisture permeability of not less than 10000 g/m$^2$·24hr, and a water resistance pressure of not less than 500 mmH$_2$O.

2.  A medical sheet base as set forth in claim 1, wherein the polyurethane nonwoven fabric and the polyurethane film are combined with each other by using an adhesive, and the adhesive is applied to parts of the polyurethane nonwoven fabric and the polyurethane film.

3.  A medical sheet base as set forth in claim 1 or 2, which has a recovery ratio of not less than 70% as measured after the medical sheet base is stretched by 100%, an air permeability of 0.01 to 1.0 cc/cm$^2$·sec, a moisture permeability of not less than 5000 g/m$^2$·24hr, and a bending resistance of not greater than 60 mm.

4.  A medical sheet base as set forth in any one of claims 1 to 3, which has an air permeability of 0.015 to 1.5 cc/cm$^2$·sec as measured when the medical sheet base is stretched by 100%.

5.  A medical sheet comprising a medical sheet base as recited in any one of claims 1 to 4.

**Patentansprüche**

1.  Medizinisches Basis-Flächengebilde, das ein Polyurethan-Vlies und einen Polyurethanfilm miteinander kombiniert umfasst,
    wobei das Polyurethan-Vlies Endlosfäden umfasst, die jeweils einen durchschnittlichen Fadendurchmesser von nicht größer als 50 $\mu$m aufweisen, wobei die Fäden in einen im Wesentlichen ungebundenen Zustand abgelegt und miteinander verbondet werden,
    wobei der Polyurethanfilm winzige Poren aufweist, die auf gegenüberliegenden Flächen des Polyurethanfilms angeordnet sind und jeweils einen Durchmesser von nicht größer als 10 $\mu$m aufweisen und in einer durchschnittlichen Distanz von nicht mehr als 10 $\mu$m voneinander beabstandet sind und winzige Poren entlang ihrer Dicke miteinander in Strömungsverbindung stehen und der Polyurethanfilm eine Luftdurchlässigkeit von 0,01 bis 2,0 cc/cm$^2$·s, eine Feuchtigkeitsdurchlässigkeit von nicht weniger als 10000 g/m$^2$·24 h und einen Wasserwiderstandsdruck von nicht

weniger als 500 mmH$_2$O aufweist.

**2.** Medizinisches Basis-Flächengebilde nach Anspruch 1, wobei das Polyurethan-Vlies und der Polyurethanfilm unter Verwendung eines Klebstoffs miteinander kombiniert sind und der Klebstoff auf Teile des Polyurethan-Vlieses und des Polyurethanfilms aufgetragen ist.

**3.** Medizinisches Basis-Flächengebilde nach Anspruch 1 oder 2, das ein Rückstellverhältnis von nicht weniger als 70 % aufweist, das gemessen wird, nachdem das medizinische Basis-Flächengebilde um 100 % gereckt wurde, und das eine Luftdurchlässigkeit von 0,01 bis 1,0 cc/cm$^2$·s, eine Feuchtigkeitsdurchlässigkeit von nicht weniger als 5000 g/m$^2$·24 h und einen Biegewiderstand von nicht größer als 60 mm aufweist.

**4.** Medizinisches Basis-Flächengebilde nach einem der Ansprüche 1 bis 3, das eine Luftdurchlässigkeit von 0,015 bis 1,5 cc/cm$^2$·s aufweist, die gemessen wird, nachdem das medizinische Basis-Flächengebilde um 100 % gereckt wurde.

**5.** Medizinisches Flächengebilde, das ein medizinisches Basis-Flächengebilde nach einem der Ansprüche 1 bis 4 umfasst.

**Revendications**

**1.** Base de drap d'examen médical comprenant un tissu non tissé en polyuréthane et un film de polyuréthane combinés entre eux,
dans laquelle le tissu non tissé en polyuréthane comprend des filaments continus ayant chacun un diamètre moyen inférieur ou égal à 50 μm, les filaments étant déposés dans un état essentiellement non lié et liés entre eux, dans laquelle le film de polyuréthane a des pores minuscules présents sur des surfaces opposées dudit film et ayant chacun un diamètre inférieur ou égal à 10 μm et étant espacés entre eux d'une distance moyenne inférieure ou égale à 10 μm, et des pores minuscules communiquant entre eux sur toute l'épaisseur dudit film, et le film de polyuréthane a une perméabilité à l'air située entre 0,01 et 2,0 cc/cm$^2$·s, une perméabilité à l'humidité supérieure ou égale à 10000 g/m$^2$·24·h, et une pression de résistance à l'eau supérieure ou égale à 500 mmH$_2$O.

**2.** Base de drap d'examen médical selon la revendication 1, dans laquelle le tissu non tissé en polyuréthane et le film de polyuréthane sont combinés entre eux à l'aide d'un adhésif, et l'adhésif est appliqué sur des parties de tissu non tissé en polyuréthane et sur le film de polyuréthane.

**3.** Base de drap d'examen médical selon la revendication 1, ayant un taux de récupération supérieur ou égal à 70 % lors de la prise de mesure après que la base de drap d'examen a été étirée de 100 %, une perméabilité à l'air située entre 0,01 et 1,0 cc/cm$^2$·s, une perméabilité à l'humidité supérieure ou égale à 5000 g/m$^2$·24 h, et une résistance à la flexion inférieure ou égale à 60 mm.

**4.** Base de drap d'examen selon l'une quelconque des revendications 1 à 3, ayant une perméabilité à l'air située entre 0,015 et 1,5 cc/cm$^2$·s lors de la prise de mesure quand la base de drap d'examen est étirée de 100 %.

**5.** Drap médical comprenant une base de drap médical selon l'une quelconque des revendications 1 à 4.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 8033673 A
- JP 2004202923 A
- JP 2004315817 A